(19) Europäisches Patentamt
European Patent Office
Office européen des brevets

(11) **EP 3 479 098 B1**

(12) **EUROPEAN PATENT SPECIFICATION**

(45) Date of publication and mention
of the grant of the patent:
**27.09.2023 Bulletin 2023/39**

(21) Application number: **17737958.3**

(22) Date of filing: **27.06.2017**

(51) International Patent Classification (IPC):
*G01N 21/552* (2014.01)   *G01N 21/85* (2006.01)
*G01N 33/28* (2006.01)   *E21B 47/113* (2012.01)
*G01J 3/02* (2006.01)   *G01J 3/427* (2006.01)
*G02B 5/12* (2006.01)

(52) Cooperative Patent Classification (CPC):
**G01N 21/552; E21B 47/113; G01J 3/021;
G01J 3/0218; G01J 3/427; G01N 21/8507;
G01N 33/2841; G01N 33/2847; G02B 5/12;**
G01N 2201/08

(86) International application number:
**PCT/US2017/039461**

(87) International publication number:
**WO 2018/005465 (04.01.2018 Gazette 2018/01)**

(54) **APPARATUS AND METHOD FOR ANALYZING COMPOSITION IN OIL AND GAS PRODUCTION WELL**

VORRICHTUNG UND VERFAHREN ZUR ANALYSE DER ZUSAMMENSETZUNG IN EINER ÖL- UND GASFÖRDERBOHRUNG

APPAREIL ET PROCÉDÉ D'ANALYSE DE COMPOSITION DANS UN PUITS DE PRODUCTION DE PÉTROLE ET DE GAZ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2016 IN 201641022386**

(43) Date of publication of application:
**08.05.2019 Bulletin 2019/19**

(73) Proprietor: **Baker Hughes Oilfield Operations, LLC
Houston, Texas 77073 (US)**

(72) Inventors:
• **MAITY, Sandip
Bangalore 560066
Kamataka (IN)**
• **KAVOORI SETHUMADHAVAN, Nagapriya
Bangalore 560066
Kamataka (IN)**

• **GHOSH, Sampa
Bangalore 560094
Karnataka (IN)**
• **MUKHERJEE, Moitrayee
Kolkata 700086
West Bengal (IN)**

(74) Representative: **Novagraaf Group et al
Chemin de l'Echo 3
1213 Onex / Geneva (CH)**

(56) References cited:
**EP-A2- 0 206 433   WO-A1-2016/044005
US-A1- 2002 176 646   US-A1- 2007 120 051
US-A1- 2012 170 023   US-A1- 2015 027 243
US-A1- 2016 164 125**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

This invention relates generally to oil and gas production wells, and more particularly to apparatus and methods for discriminative detection of constituents in a mixed phase medium, present in an oil and gas production well, required for controlling and planning the production from the oil and gas production well.

US 2015/027243 A1 discloses a probe for analysing different phases of an oil/ water/gas mixture in a downhole, the probe comprising multiple reflective surfaces interacting with a fluid in the downhole.

US 2016/164125 A1 discloses an optical probe comprising multiple (e.g. three) points of internal reflection, which is used for analysing concentrations of different compounds in an electrolyte cell by measuring absorption/evanescent coupling at different wavelengths, each wavelength being absorbed by one of the compounds.

US 2007/120051 A1 discloses in-situ imaging of different constituents of a fluid in a downhole by an optic probe measuring absorption at different wavelengths, and shows absorption spectra referring to absorption wavelengths of, e.g. water, oil, methane.

US 2002/176646 A1 discloses the determination of oil, water and gas in a three-phase flow in a downhole by an optical probe comprising a sapphire body having three reflection faces interacting with the surrounding fluid, e.g. a corner cube.

US 2012/170023 A1 discloses using a mid-IR spectrometer for detecting trace amounts of oil in water-based drilling mud.

WO 2016/044005 A1 discloses a sensor for monitoring $CO_2$ in a fluid comprising different phases of water, oil and gas, the sensor including an internal reflection window for contacting with the fluid, wherein a mid-infrared light beam is internally reflected at an interface between the window and the fluid and then passed through three narrow bandpass filters transmitting mid-infrared radiation bands of wavelengths corresponding to absorbance peaks of water, oil and $CO_2$.

The mixed phase medium present in any well bore that is drilled for the oil and gas production plant may have a mix of different constituents, including oil, water, gas, and other hydrocarbons, present at varying depths of the well bore. These wells may have a depth of 15,000 meters, and more. At these depths, temperatures and pressures are high and present harsh environmental conditions for material and human interventions, that may be required to determine what is the mix of constituents in the mixed phase medium of the reservoir, for production planning purpose. For example, pressure in the well may exceed fifteen-thousand to twenty-thousand (15,000-20,000) pounds per square inch (103000 - 138000 kPa) and the temperature may exceed one-hundred-eighty (180) degrees Celsius.

Typically testing tools, often referred as formation testing tools or well bore testing tools or production logging tools, are used for conducting different tests to assess different parameters needed for assessment of the oil and gas production well, including testing of mixed phase medium. Many of these tools are suspended into the well bore and obtain fluid samples from the well bore, and conduct spectral analysis on the collected samples to determine the constituents in the fluid sample, and predict performance of the well. The fluid samples in some cases are brought to a surface testing unit, or alternately, into a chamber in the tool, where further analysis is conducted. These tools and the associated methods, suffer from an intrinsic drawback that the collected sample is often contaminated by the drilling fluid and other contaminants that appear during the process of drilling the well bore. Further removal of the sample from the home environment of high pressure and temperatures, often changes the properties of the sample being stored in a chamber or in the surface testing unit, and therefore the results from such testing tools and methods do not represent an accurate assessment of the constituents of the reservoir. Still further, since the mixed phase medium in the well flows in from the earth formation around the well, it is usually in a mobile flow, and therefore removal of the sample does not give the present composition of the mixed phase medium, and any production planning decisions that are derived from such tests will not be accurately responding to the present composition of the mixed phase medium. This may therefore lead to sub-optimal performance of oil and gas well.

To overcome this issue, some tools and techniques have been created for in-situ measurement of the mixed phase medium. For example, in one technique, a tool is used that allows light in the visible and near infra red (IR) region to be transmitted through the fluid sample directly in the well. A spectrometer present in the tool body measures the spectrum of the transmitted and die back scattered light from the fluid sample, and knowing the spectrum of the incident light, transmission and backscattered light, absorption spectra for the sample are determined. Then using absorption spectra of water, gas, crude and refined oils, and drilling fluids, a least squares analysis based on known analytical computation methods is performed that models the observed spectra as a weighted sum of the spectra of its components, the least squares analysis giving the composition of the fluid in terms of weights of the various components.

The above mentioned technique, referred also as transmission-absorption technique, or transmission technique, requires large path lengths through the sample that are necessary to get detectable measurements, being typically 5 mm. or more. This leads to complexity in instrumentation, and even at this length of transmission, signal levels are lower and the spectral analysis of the harmonics and combinations is complicated. Also of importance is the fact that in well bore, the presence of particulate matter, microscopic particles or bubbles leads to scattering. This scattering drastically increases the optical density and reduces the ability to detect spectral features of the sample.

Some other tools and methods have employed use of diffuse and specular reflectance methods for near infrared analysis of a sample in the well bore. Diffuse reflectance measurements require a large solid angle of data collection with a relatively large illuminated area to average out sample in-homogeneities. Consequently, diffuse reflectance is not suitable for measurement of small samples at low flow rates that are characteristic of reservoir fluid monitoring.

For example, one of the existing methods describes detecting the presence of gas in a flow stream that comprises oil, water, gas or particulates within the borehole. The apparatus comprises a flow line for containing the fluid and a light source for transmitting light towards the fluid in the flow line. A prism transmits light from the source to the fluid and forms an interface with the flow line. The interface reflects the light from the source and a detector array detects the light. The angle of incidence at which total reflection of the light takes place provides a measure of the refractive index of the fluid in contact with the prism surface. As the refractive index of gas and liquids are essentially different, the amount of gas in the fluid can be measured. Specular reflectance methods are effective in identifying a gas phase in the composition of a multiphasic fluid but are not particularly useful for identifying water and oil phases. Further, even for the gas phase detection, there is a possibility of inaccuracy because at sufficiently high pressures, the refractive index of the gas phase approaches the refractive index of liquid hydrocarbons, and specular reflectance methods cannot be used to determine methane concentration at high pressures.

Some of the other proposed tools and techniques for in-situ testing are based on attenuated total reflection (ATR) which collects a reflected evanescent wave from the mixed phase medium and analyses it to determine whether the signature being carried is that of gas, in particular, methane, or liquid (i.e. only single phase, or two phase discrimination). ATR methods make use of the fact that when light is incident at an interface between a first medium of a higher refractive index and a second medium of lower refractive index, there exists a critical angle beyond which total reflectance of the light takes place within the first medium. However, even beyond the critical angle, an evanescent wave is propagated into the second medium with a characteristic depth of penetration $dp$ into the second medium given by

$$d_p = \frac{\lambda}{2\pi n_{21} \sqrt{\sin^2\theta - n_{21}^2}}$$

where n 21=n2/n1, the ratio of the refractive indices of the second and first media and $\lambda$ is the wavelength of the radiation in vacuum and the angle $\theta$ exceeds the critical angle.

The energy carried by this evanescent wave manifests itself as a decrease in the energy of the reflected wave, a measurable quantity. ATR methods thus effectively function like the equivalent of a transmission cell having a transmission length de given by $de=0.5\,(d_s+d_p)$ where $d_s$ is given by:

$$d_s = \frac{n_{21}\lambda\cos\theta}{\pi n_1(1 - n_{21}^2)\sqrt{\sin^2\theta - n_{21}^2}}$$

where $d_s$ is the effective thickness for light polarized perpendicular to the plane of incidence and $d_p$ is given by

$$d_p = \frac{n_{21}\lambda\cos\theta}{\pi n_1(1 - n_{21}^2)\sqrt{\sin^2\theta - n_{21}^2}\,[(1 + n_{21}^2)\sin^2\theta - n_{21}^2]}$$

Where $d_p$ is the effective thickness for light polarized in the plane of incidence. Some calculations will show that the effective thickness is of the order of the wavelength of the light.

One of the prior art tool describes an optical probe that carries an internal reflectance crystal and is in contact with the fluid in the reservoir. An acousto-optical tunable filter (AOTF) transmits a single wavelength of light from a broad band light source, that is carried by an optic fiber to the internal reflectance crystal where it undergoes total reflection at the crystal faces in contact with the fluid. An evanescent wave propagates into the fluid with a depth of penetration related to the absorption of the light in the fluid. The reflected light carries information about this absorption which is determined by the chemical composition of the fluid. 2. return optical fiber conveys the reflected light back to a spectrometer. A processor determines the absorption at the wavelength of the monochromatic light by comparing the energy in the reflected light to the energy in the incident light. By repeating this process at a number of different wavelengths, the absorption spectrum of the fluid is determined. Principal component analysis or least squares analysis of this absorption spectrum using known absorption spectra of constituents likely to occur in the fluid gives the composition of the fluid in terms of these constituents.

In some embodiments of prior art, like the one described herein above, a prism made of sapphire is used as a reflector to achieve total internal reflection and evanescence is collected from the tip of the prism that is exposed to reservoir fluids. However, since the refractive indices of the sapphire and mixed phase medium, as mentioned herein above, are very close, the evanescence (which to begin with is a very low energy signal) will be very weak, in such reflector structures, and possibly all light will be refracted in the mixed phase medium, when the mixed phase medium is primarily oil. Hence such methods are likely unreliable to accurately determine all the phases of the mixed phase medium.

Thus the current tools and techniques suffer from low signal detection since the evanescent wave has low energy, and its detection with the totally internally reflected signal poses a challenge as explained herein above. Further, these kind of in-situ tools require precision in the instrumentation needed to shine light at a considerable depth within the earth's surface and carry the reflected signal back for analysis. These requirements pose further challenges for obtaining direct measurements from the mixed phase medium present in the oil and gas production wells.

BRIEF DESCRIPTION

The present invention is defined in claims 1 and 10. In one aspect, an apparatus (12) for analyzing a composition of mixed phase medium (14) present in a reservoir (16) at a test site (18) of an oil and gas production well (20) is provided. The mixed phase medium (14) comprises gas, water and oil. The apparatus includes multiple optical sensor probes (26) suspended at one end of a wireline (24), and each optical sensor probe (26) generates a characteristic output (86) representing the composition of mixed phase medium (14) tested by the respective optical sensor probe (26). A surface processing and display unit (28) is connected at an other end of the wireline (24) for receiving the characteristic output (86) from each of the plurality of optical sensor probes (26).

Each of the plurality of optical sensor probes (26) include an optical circuitry (42) for generating guided incident light (48) having more than one selectable wavelengths (46) of light that impinge the mixed phase medium (14) simultaneously, and for receiving collected light (54) from the mixed phase medium (14). The more than one selectable wavelengths (46) include at least a first selectable wavelength substantially absorbed by oil, and substantially reflected by gas and water, and a second selectable wavelength substantially absorbed by water and substantially reflected by gas and oil.

A multi-facet reflector (56) is provided in the optical sensor probe (26) and is in contact with the mixed phase medium (14) at the test site (18), for receiving the guided incident light (48) that travels on a triangular path (50) in the multi-facet reflector (56) and impinges the mixed phase medium (14) at a plurality of interaction points (66), and returns as the collected light (54) from the mixed phase medium (14) at the plurality of interaction points (66).

Optical sensor probe also includes an optical fiber (52) coupled to the optical circuitry (42) and the multi-facet reflector (56), where the optical fiber (52) receives the guided incident light (48) from the optical circuitry (42), transmits the guided incident light (48) to the multi-facet reflector (56), receives the collected light (54) from the multi-facet reflector (56) and transmits the collected light (54) to the optical circuitry (42). In this arrangement, a principal axis (60) of the optical fiber (52) aligns substantially with a central axis (58) of the multi-facet reflector (56),

The optical circuitry (42) converts the collected light (54) into respective electrical signals and processes the respective electrical signals to generate the characteristic output (86) representing the composition of the mixed phase medium (14) at the test site (18).

In another aspect, a method for analyzing *in-situ* a composition of mixed phase medium (14) present in a reservoir (16) at a test site (18) of an oil and gas production well (20), where the mixed phase medium comprises gas, water and oil, is presented.

The method includes steps for generating more than one selectable wavelengths of light; impinging the more than one selectable wavelengths of light simultaneously as guided incident light on a multi-facet reflector placed in the mixed phase medium, where die guided incident light is capable of travelling in a triangular path in the multi-facet reflector; and transmitting the guided incident light through the multi-facet reflector, such that the guided incident light impinges the mixed phase medium at a plurality of interaction points travelling on the triangular path in a forward direction and a return direction. From the return direction, collected light from the plurality of interaction points is received ; and electrical signals representative of collected light are detected. Processing of these electrical signals is done to generate a characteristic output representative of composition of the mixed phase medium. As mentioned herein above, the more than one selectable wavelengths include at least a first selectable wavelength substantially absorbed by oil, and substantially reflected by gas and water, and a second selectable wavelength substantially absorbed by water and substantially reflected by gas and oil.

DRAWINGS

These and other features, aspects, and advantages of the present invention will become better understood when the following detailed description is read with reference to the accompanying drawings in which like characters represent like parts throughout the drawings, wherein:

FIG. 1 is an exemplary arrangement showing an apparatus for analyzing a composition of mixed phase medium present at a test site of a vertical oil and gas production well;
FIG. 2 is another exemplary arrangement showing an apparatus for analyzing a composition of mixed phase medium present at a test site of a horizontal oil and gas production well;
FIG. 3 is a diagrammatic representation of optical sensor probes used in the apparatus of FIG. 1 and FIG. 2;
FIG. 4 is a block diagram representation of apparatus of FIG. 1 and FIG. 2 to explain the working of the apparatus;
FIG. 5 is a cross-sectional representation of an exemplary embodiment of a multi-facet reflector used in the optical sensor probes of the apparatus of FIG. 1-FIG. 4;
FIG. 6 is a top view of the exemplary multi-facet reflector of FIG. 5;
FIG. 7 is a diagrammatic representation of some examples of the multi-facet reflector; and
FIG. 8 is a flowchart representation of exemplary steps for a method for analyzing a composition of a mixed phase medium that includes gas, water, and oil, present in a reservoir at a test site.

DETAILED DESCRIPTION

In oil and gas production plants, production planning and operation requires periodic testing of a reservoir where an oil and gas production well is located, to reliably know mix of constituents of a mixed phase medium that is present at varying depths of the reservoir. The mix of constituents of the mixed phase medium referred herein includes oil, gas and water, that are referred collectively as 'constituents' or individually as a corresponding 'phase' of the mixed phase medium. Alternately, it may be desirable to assess the mix of different constituents in the reservoir for characterization of the oil and gas production well as "essentially oil" or "essentially gas", or an indicator for "mix of oil and gas". Still further, it may be desirable to assess the mix of different constituents in the reservoir for assessing the remaining life of an existing oil and gas production plant. The mix of the constituents, may be further used to determine the capacity of the oil and gas production plant, associated machinery required, and other such production planning, control and operation decisions.

The apparatus and method described herein that are used for determining the composition of the mixed phase medium, advantageously allow for simultaneous discrimination between all the three phases, oil, gas and water, instead of the usual fluid only or gas only discrimination offered in the prior art. Further, the apparatus and method described herein, provide an ability for conducting these tests directly on a test site of the oil and gas production well, with speed, accuracy, and greater reliability. 'Test site' referred herein is a region at a desirable depth of the oil and gas production well which is selected for testing of the mixed phase. The depth is determined based on prior knowledge of the well bore in the oil and gas production plant. Further, the apparatus and method described herein work for both horizontal and vertical well bores.

Aspects of the invention are described herein below in more detail in reference to the drawings.

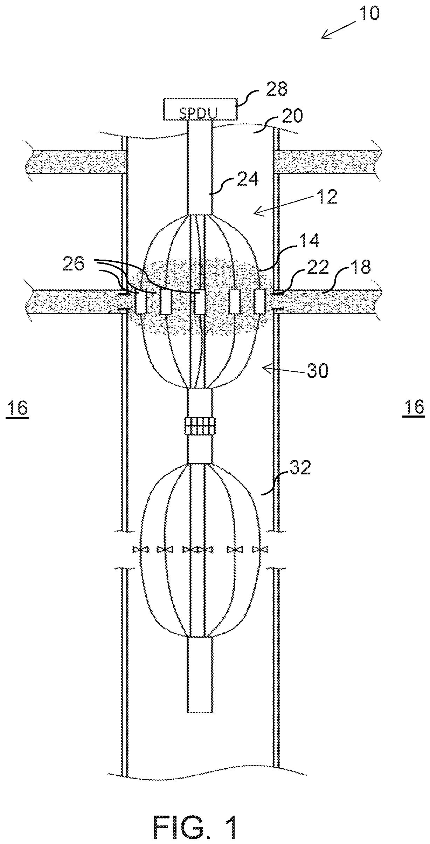

FIG. 1 shows the schematic arrangement 10 of an apparatus 12 used for analyzing the composition of a mixed phase medium 14 present in the reservoir 16 at the test site 18 of a vertical oil and gas production well 20 ('well' mentioned herein refers to a well borehole). The test site 18 is typically situated at a location of production valve 22 that allows flow of mixed phase medium into the well 20. A wireline 24 which is typically a multi conductor cable, is used for suspending multiple optical sensor probes 26 of apparatus 12, at one end of the wireline 24 inside the well 20. The other end of the wireline 24 is connected to a surface processing and display unit (SPDU) 28 of the apparatus 12.

The multiple optical sensor probes 26 may be arranged in different configurations, such as, but not limited to the configuration 30, referred generally as a bowstring configuration 30, shown in FIG. 1, for suspending the optical sensor probes 26 from the wireline 24, and anchoring them in close proximity to the test site 18.

The multiple optical sensor probes 26 are constructed such that the outer casing and internal components of each multiple optical sensor probe 26 is relatively impervious and resistant to the harsh conditions of the reservoir 16 and well 20. In a non-limiting example, the multiple optical sensor probes 26 may withstand pressure in a range of about fifteen-thousand to twenty-thousand (15,000-20,000) pounds per square inch (psi) (103000-138000 kPa) and a temperature range of about 180 degrees Celsius to 200 degrees Celsius. The casing material for the multiple optical sensor probes 26 may be titanium, stainless steel, Inconel, brass, or the like.

The optical sensor probes 26 of apparatus 12 referred herein are used to obtain the composition of the mixed phase medium being tested, in terms of phase fraction of each of the phases of the mixed phase medium. As shown in FIG. 1, the apparatus 12 may also include a spinner 32 to determine at least a velocity of the mixed phase medium 14 in the well 20. Both these measurements, i.e. the phase fractions and the velocity, are then used to determine the GLR i.e. gas to liquid ratio, more accurately, at the test site. The GLR thus obtained is further used for production planning and control actions.

The optical sensor probes 26 may be withdrawn from the well 20 by known methods. The optical sensor probes 26 may also be moved to another position in the well at which the analysis of the mixed phase medium is to be performed.

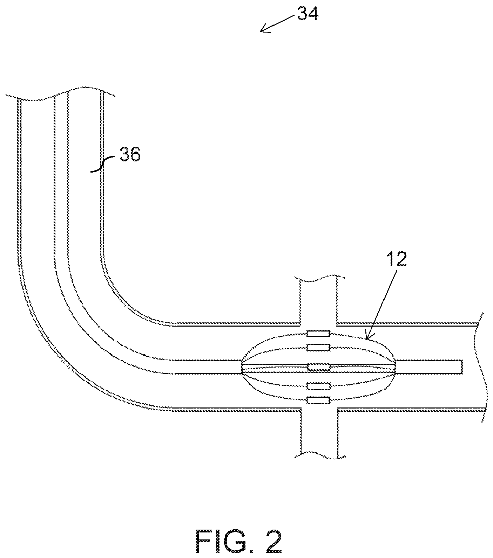

While FIG. 1 refers of an arrangement of apparatus 12 in a vertical well 20, FIG. 2 presents a diagrammatic representation 34 of a horizontal well 36 with the apparatus 12. As would be understood by those skilled in the art, in this case the test site 18 will be at varying lengths of the well. Thus the apparatus 12 is suitable for both vertical and horizontal wells, as shown in FIG. 1 and FIG. 2 respectively.

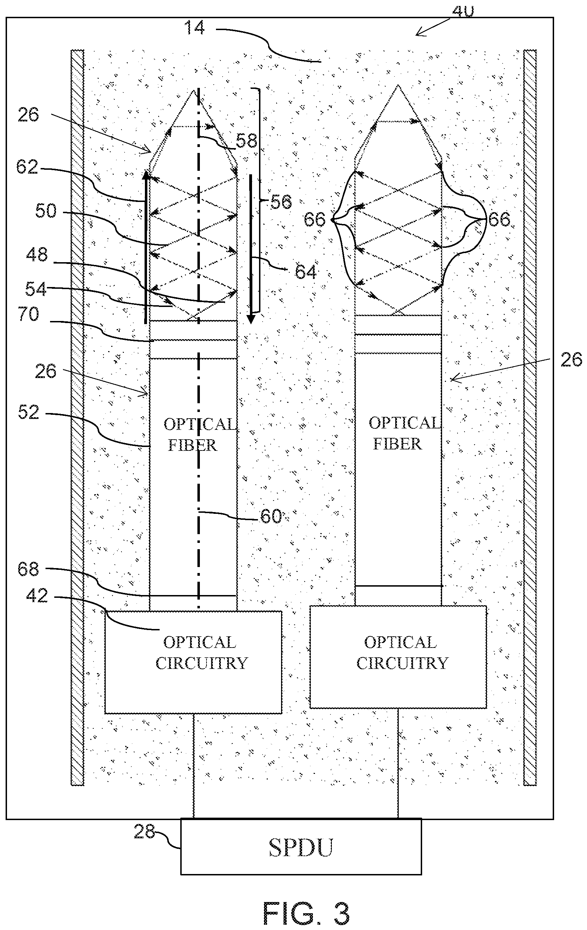

Now turning to FIG. 3, a diagrammatic representation 40 is presented to show two of the optical sensor probes 26 referred in FIG. 1 and FIG. 2, that are used to test the mixed phase medium 14. As shown, each optical sensor probe 26 comprises an optical circuitry 42 for generating guided incident light 48 of more than one selectable wavelengths. The guided incident light 48 travels through an optical fiber 52 and then through a multi-facet reflector 56 on a triangular path 50 (triangular path 50 is depicted by the dotted arrows inside the multi-facet reflector 56).

The optical circuitry 42 also receives through the optical fiber 52, collected light 54 reflected back from the mixed phase medium 14 through die multi-facet reflector 56. The optical fiber 52, in one example may have a diameter in the range of about 280 microns to about 310 microns. In a non-limiting example, the length of the optical fiber 52 is about 2 feet. The length of the optical fiber may depend upon the configuration of apparatus 12 of FIG.1 and 2. In certain embodiments, the optical fiber 52 may have a coating of carbon, hydrogen capturing gels, or the like to prevent formation of hydroxyl due to the presence of free hydrogen atoms inside the well.

The more than one wave selectable wavelengths referred herein include at least a first selectable wavelength substantially absorbed by oil, and substantially reflected by gas and water, and a second selectable wavelength substantially absorbed by water and substantially reflected by gas and oil. Wavelengths of about 350 nm (nano meters) to 450 nm, present a range of wavelengths that are substantially absorbed in oil, and therefore a desired wavelength in this range may be selected for discrimination of oil phase. In one exemplary embodiment, a wavelength of 405 nm was selected that is substantially absorbed in oil. Similarly wavelengths of about 1.2um (micro meter) to about 3.3 um, present a range of wavelengths that are substantially absorbed in water, and therefore a desired wavelength in this range may be selected for discrimination of water. In one exemplary embodiment, a wavelength of 3um was selected that is substantially absorbed in water. It would be appreciated by those skilled in the art that simultaneous interrogation of the mixed phase medium 14 using the selectable wavelengths simultaneously as mentioned herein, removes several errors that are present due to an uncertainty that arises when only single wavelengths are used or when multiple wavelengths are used but are not synchronized to impinge the mixed phase medium simultaneously. This is due to the fact that the mixed phase medium is continuously in motion and any time delay based techniques do not accurately capture the composition of mixed phase medium accurately.

It would also be worthwhile to note here that only a single optical fiber 52 is used for both guided incident light 48 and collected light 54, thus simplifying the transmission and reception process. This further adds to simplification of the optical sensor probe 26 due to reduction of physical components required for conducting such tests, which is also a critical requirement in these applications, where testing ideally is required to be conducted within very space constrained regions.

Reverting back to FIG. 3, the multi-facet reflector 56 is used for receiving the guided incident light 48 from the optical fiber 52 and for transmitting to the optical fiber 52, the collected light 54 from the mixed phase medium 14. Here it may be worthwhile to note that a principal axis 60 of the optical fiber 52 aligns substantially with a central axis 58 of the multi-facet reflector 56 for focused transmission of the guided incident light in the multi-facet reflector 56, and reception of collected light at the optical fiber 52. The guided incident light 48 travels over the triangular path 50, traversing an entire length of the multi-facet reflector 56, first in a forward direction 62, and then in a return direction 64 (as it would be clear from the drawing, both forward and return directions follow a triangular pattern). During this travel, or propagation of the guided incident light 48, the more than one selectable wavelengths that it carries impinge the mixed phase medium 14 simultaneously at multiple interaction points 66 spread over a surface of the multi-facet reflector 56 (shown as the tip of arrowheads in reflector 56).

At each of the interaction points 66, the guided incident light 48 undergoes either one or more of total internal reflection, and evanescence absorption of selected wavelengths, depending on the composition of mixed phase medium 14. Thus at each of the interaction points 66, one or more reflected wavelengths of light, including any evanescence waves from the mixed phase medium, are received as collected light 54, and also travel further over the triangular path 50 with the guided incident light 48 in the entire length of the multi-facet reflector 56 to reach the optical fiber 52 as collected light 54 for further processing. Such propagation of light ensures multiple points of interactions with the mixed phase medium 14, in forward and return direction, and allows for increased sensitivity of the signal that is obtained from the collected light 54. In other words, by virtue of number of interactions and the guided wave path (triangular path), the collected light 54 carries more accurate information of the characteristics of the mixed phase medium 14.

For example, in an exemplary implementation, wavelengths of 3um and 405nm were used. It is known that 3um wavelength is completely reflected in gas and oil at a given angle of incidence, and substantially absorbed in water, and reflects an evanescent signal in water under total internal reflectance conditions. Thus if the analysis of collected signal shows an attenuated wavelength for 3um, then water is present, if no change happens to 3um also, then only gas is present. Similarly 405nm is substantially absorbed in oil, and reflected in gas and water. Thus if the collected wavelength shows an attenuated wavelength of 405nm, then the mixed phase medium comprises oil. If the collected signal shows 3um and 405 nm with attenuation, then the mixed phase medium comprises gas, water and oil. Alternately, if the collected wavelength comprises 3um and 405 nm without attenuation signals, then only gas is present. Further, when the determination is that oil is present, the evanescence signal from oil can be easily determined from the collected light, and further analyzed to determine what type of oil signature. The concentration of oil in the mixed phase medium can also be determined, by known spectral analysis. Similarly the concentration of water in the mixed phase medium can be determined by analyzing the evanescence signal in case of determination that water is presentation. Thus by using more than one selectable wavelengths, discrimination between all three phases can be achieved. The present invention can therefore determine directly phase fractions without the need for complex calculations and empirical formulae.

The above referenced analysis results are obtained as characteristic output from the optical circuitry 42 of the optical sensor probe 26, and are communicated to the surface processing and display unit 28 (SPDU), for use by an operator or a controller for further actions, for controlling production by opening or closing the production valves of the oil and gas well through different control actions. In some exemplary embodiments a controller (not shown in FIG.3) is assigned for each well. In some other embodiments, a central controller is deployed that is responsible for all the wells in a defined area of the oil and gas production plant. It may be understood by those skilled in the art that the surface processing and display unit 28 (SPDU), may be used as the controller referred herein in some embodiments, and in some other embodiments the control functions may be embodied in a separate processing and computing machine. The control actions referred herein may include, controlling water flooding or steam injection in the well.

The surface processing and display unit 28 in some embodiments may include instructions for further processing of the characteristic output from each of the optical sensor probes 26 to provide an overall discrimination of the composition of mixed phase medium 14 at the test site 18. It would be understood by those skilled in the art that the surface processing and display unit 28 is in operational communication with the multiple sensor probes 26. In the exemplary embodiment, the surface processing and display unit 28 is in a physical communication with the device 16 via a wire or wireless means. Further, a user (not shown) may view the analysis results using a user interface of the processing and display unit 28. For example, a user may view the presence/absence or concentration of the different phases of the mixed phase medium 14.

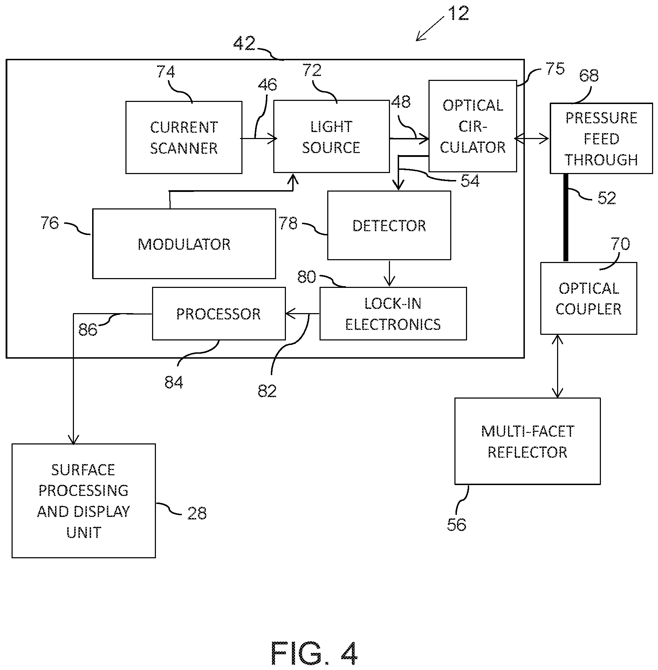

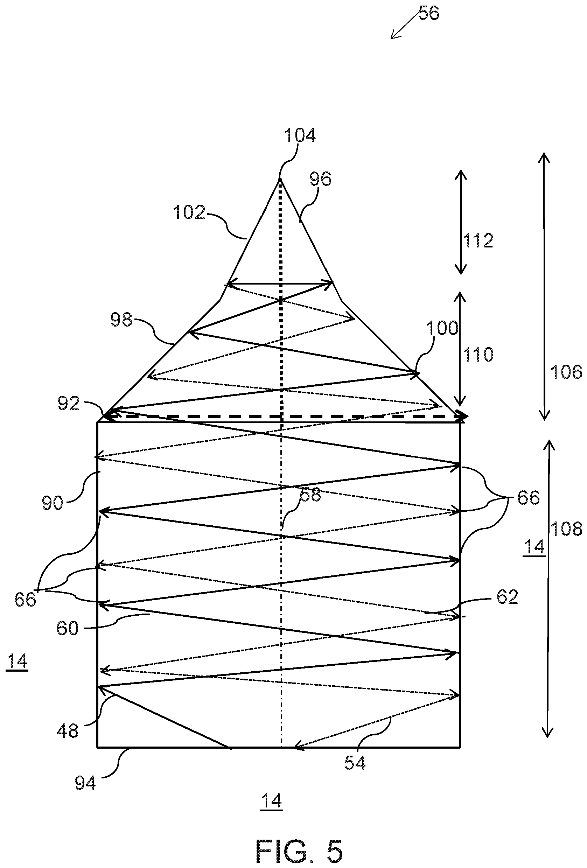

Also shown in FIG. 3 is a pressure feedthrough 68that provides a barrier for high pressure, to the optical circuitry 42. An optical coupler70 is also used in the exemplary embodiment so that there is no scattering of either the guided incident light 48 while leaving the optical fiber 52 to impinge the multi-facet reflector 56, and the collected light 54 while entering the optical fiber from the multi-facet reflector 56. Both the optical circuitry 42 and the multi-facet reflector 56 are further described to understand the working of the invention, in reference to subsequent description in reference to FIG. 4 and FIG. 5.

FIG. 4 is a block diagram representation of apparatus 12 for further explaining the working of the invention. The optical circuitry 42 as shown in FIG. 4 includes a current scanner 74 that provides an input of the variable current so that more than one selectable wavelength of light 46 to a light source 72 for generating these more than one selectable wavelengths of light simultaneously as guided incident light 48. The choice of the more than one selectable wavelengths of light is user determined in the exemplary embodiment. It would be understood by those skilled in the art that the guided incident light 48 is directed to impinge the multi-facet reflector 56 at an angle of incidence that will allow total internal reflectance of the more than one selectable wavelengths of light at the multiple interaction points located on a surface of the multi-facet reflector, and on the triangular path in the multi-facet reflector.

The light source 72 is at least one of a coherent source, or an incoherent source. In one exemplary implementation, the coherent source is a distributed feedback laser with current and temperature tuning of variable wavelength that allows for generating different selectable wavelengths of light. In another exemplary embodiment, where the incoherent source is used, it is implemented using a linear variable filter for continuous wavelength selection for generating the more than one selectable wavelengths of light.

The optical circuitry 42 in an exemplary implementation also includes a modulator 76 for modulating the wavelengths of the guided incident light 48 that is generated from the light source 72, and use of heterodyne lock-in technique, which mixes a frequency different from a frequency of the signal represented as incident light 48, with each of the selectable wavelengths in the incident light 48 for modulation, and during de-modulation, signals reflected only due to the impact of the selectable wavelengths of light can be easily detected, in comparison of all other noise signals that may travel with the reflected light 54 that impinges a detector 78. In one exemplary implementation, each of the selectable wavelength is modulated with different frequency modulation frequency, for example, the selectable wavelength 3um is modulated with a frequency of 10Khz, and selectable wavelength 450nm is modulated with a frequency of 20KHz. Generally, the modulation is achieved by known techniques such as wavelength modulation spectroscopy.

The optical circuitry 42 also includes the optical coupler 70 for coupling the optical fiber with the multi-facet reflector 56 as mentioned in reference to FIG. 3. In one embodiment die optical coupler 70 is a spherical lens assembly, such as a graded refracted index lens (GRIN). This is used to overcome a difference in numerical aperture of the optical fiber 52 and the multi-facet reflector 56. For example, the optical fiber 52 may have a numerical aperture of 0.22, and the multi-facet reflector 56 may have a numerical aperture of 0.87. The difference in apertures, would result in scattering of collected light when returning into the optical fiber. Thus in order to ensure that the there is no loss in transmission or reception due to difference in numerical apertures of the optical fiber 52 and multi-facet reflector 56, the optical circulator 76 is used.

The optical circuitry 42 includes a detector 78 for receiving the collected light 54 from the optical fiber 52, and for generating a detector output 82 which are electrical signals representative of wavelengths present in the collected light 54. The detector 78 in one implementation uses frequency multiplexing lock-in electronics 80 that allows separation of each wavelength signal in the collected light 54 by heterodyne lock-in technique explained in reference to the modulator 76. If for example, each of the wavelengths was modulated with different frequency modulation frequency as referred herein above, 3um: 10Khz, 450nm-20KHz, individual wavelength signals from the detector are obtained by heterodyne demodulation that picks-up the reflected signals travelling on frequencies of 10Khz and 20KHz as detector output 82. An optical circulator 75 is also used in the exemplary embodiment so that the incident light 48 guides through the optical fiber 52 from light source 72 to the multi-facet reflector 56 and the reflected light 54 goes back to the detector 78 only, and does not enter light source 72. It would be appreciated by those skilled in the art that if reflected light 54 goes back to the light source 72, it would cause instability for the guided incident light 46.

The optical circuitry 42 further includes a processor 84 for analyzing the detector output 82 and using it to determine the composition of the mixed phase medium being tested. The characteristic output 86 from processor 84 provides the discrimination of the three phases in the mixed phase medium at the test site. More specifically, the characteristic output 86 is indicative of presence or absence or percent presence of each of gas, water and oil in the mixed phase medium.

The processor 84 may include a simple look-up table to match the detector output 82 with the wavelength signatures in the incident light 48, and determine the constituents of the mixed phase medium based on this comparison. Further details about the mixed phase medium may be found, by making measurements of the amplitude of the guided incident light 48 and of the collected light 54 from the detector output 82, and the amount of wavelengths absorbed by the mixed phase fluid at the frequency of the incident light may be determined. This is further used to determine the percentage constitution of the mixed phase medium discriminating the percentage of water, oil and gas in the mixed phase medium.

It would be appreciated here, that since the apparatus 12 comprises multiple optical sensor probes 26, it is able to simultaneously investigate the mixed phase medium at different positions in the test site simultaneously.

The multi-facet reflector 56 referred herein above is completely immersed in the mixed phase medium 14 and optically coupled to the optical fiber 52. An exemplary embodiment the multi-facet reflector 56 is shown in more detail in FIG. 5, and described herein below.

FIG. 5 is a cross-sectional representation of the multi-facet reflector 56 referred herein above in the embodiments of the invention. The multi-facet reflector 56 includes a cylinder 90 with a top end 92, a bottom end 94, and a cap 96 projecting from at least at the top end 92 of the cylinder 90, wherein the cap 96 has three or more non-similar faces 98, 100, 102 meeting at a common point 104. In one exemplary embodiment, the common point 104 lies on the central axis 58 of the cylinder 90 (also referred as the central axis of the multi-facet reflector 56), and at a predefined distance 106 from the top end 92 of the cylinder 90.

As shown in FIG.5, the guided incident light 48 impinges the multi-facet reflector 56 from the bottom end 94 of the cylinder 90, interacts with the mixed phase medium 14 at the plurality of interaction points 66 as mentioned herein above, by travelling through a length 108 of the cylinder 90 and at least a length 110 of the cap 96 on a triangular path 50 in both forward direction 62 and return direction 64.

The exemplary embodiment of FIG. 5 also shows that the cap 96 includes a tiered structure with at least two tiers, characterized by heights or distances 1 10 and 112, and wherein the first faces 98, and 100 are arranged as a first tier, and a second face 102 (there could be more faces), is arranged along a second tier, with the first tier i.e. faces 98 and 100 being positioned below the second tier. Further, the first face 98 in first tier is angularly positioned with respect to a second face 102 in the second tier. In an exemplary embodiment, the first face is positioned below the second face with angle ratio of 1:0.98 to get reflectance of up to about 30% from similar refractive index of mixed phase medium and multi-facet reflector 56.

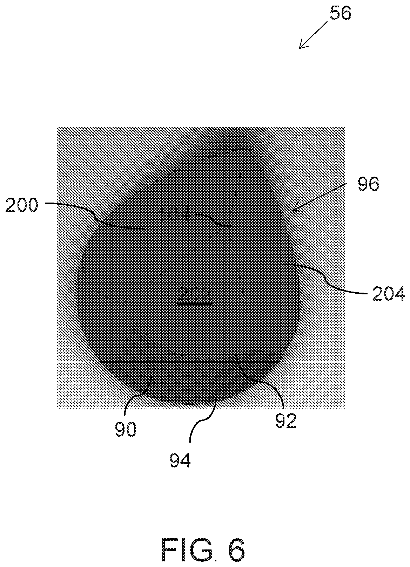

FIG. 6 is a top view of another example of a multi-facet reflector 56. The cap 96 comprises non-similar faces 200, 202, 204 arranged over the top end 92 of the cylinder 90 having the bottom end 94.

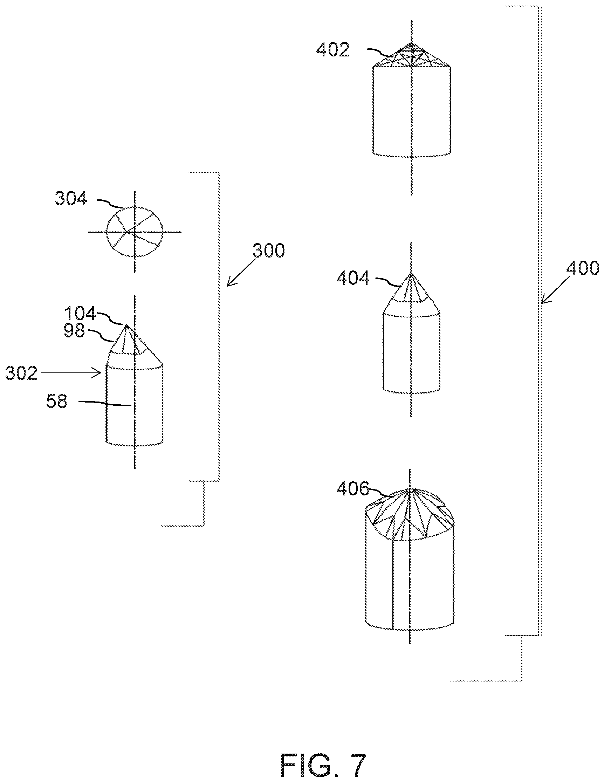

FIG. 7 is a diagrammatic representation of some other examples of the multi-facet reflector 56. The embodiment 300 is an off-centered embodiment of the multi-facet reflector 56. Off-centered referred herein means that the tip or common point 100 of the non similar faces 98 does not fall on the central axis 58 of the embodiment 302. A top view 304 of the embodiment is presented for further clarity for an off-centered embodiment of the multi-facet reflector. The other embodiments in FIG. 7, shown collectively by reference numeral 400, and individually by reference numerals 402, 404, and 406 show exemplary implementations with different faces arranged in a centered configuration, similar to FIG. 5 embodiment.

It would be appreciated by those skilled in the art that the non-similar faces, the tiered structure, and the off-centering of the tip of the multi-facet reflector 56 referred herein, offers the advantage of higher reflectance and larger area for more evanescence interaction in the mixed phase medium.

The multi-facet reflector 56 referred herein is typically an internal reflectance crystal having a high refractive index in the infra-red (IR) region of light, is chemically resistant and mechanically strong. Sapphire is used in a preferred embodiment of the invention. Sapphire is also transparent in the near and mid IR region, chemically resistant, mechanically hard and relatively inexpensive. This makes it the most cost-effective for downhole applications. Sapphire has a refractive index of 1.74. The mixed phase medium 14 referred herein may have refractive indices up to 1

Cubic zirconia that has characteristics comparable to those of sapphire may also be used. In yet another embodiment of the invention, diamond may be used. Diamonds are more expensive than sapphires or cubic zirconia, but have the advantage of a higher refractive index, transparency and extreme hardness.

The invention, which is defined in claims 1 and 10, particularly overcomes the problem posed by prior art embodiments described herein where tip of the prism is used for total inter reflection and evanescence. By deviating from these prior art embodiments, the invention increases the number of points of interactions by guiding the incident light on the triangular path, and therefore the collected signal becomes stronger at each point of interaction on the triangular path, till the light reaches back the optical fiber. Further, in complete contrast to some prior art embodiments, in a specific example, which is not in accordance with the present invention, only the cylinder part of the multi-facet reflector is exposed to the mixed phase medium, and there are no interaction points in the cap of the multi-facet reflector, the incident and collected wave still traverse the length of the cylinder and a part of the cap, and returns to the optical fiber.

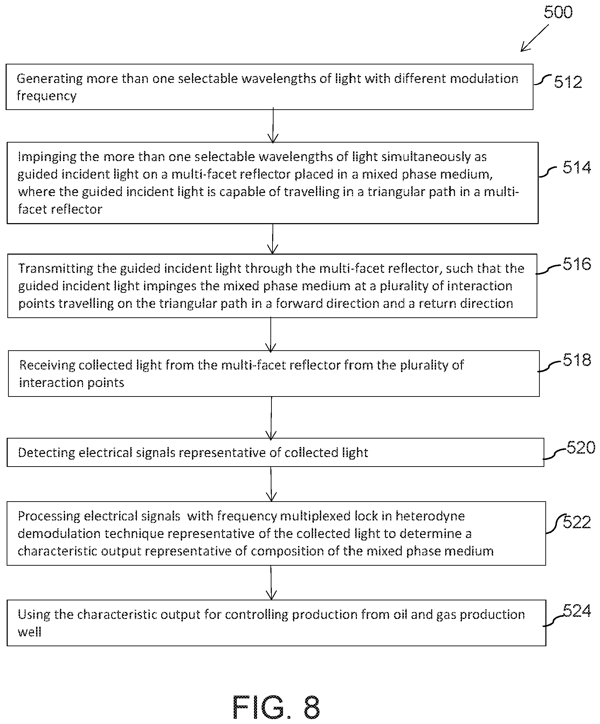

In another aspect, a method for analyzing a composition of mixed phase medium that includes gas, water, and oil, present in a reservoir at a test site of an oil and gas production well is provided, which is defined in claim 10. The method steps are summarized in a flow-chart 500 of FIG. 8, the details of these steps have already been described in relation to the working of the invention described herein above.

Referring to FIG. 8, the method includes a step 512 for generating more than one selectable wavelengths of light. A light source, either coherent or incoherent is used for generating more than one selectable wavelengths of light. At step 514, these more than one selectable wavelengths of lights is impinged simultaneously as guided incident light capable of travelling in a triangular path in a multi-facet reflector. The triangular path ensures that the incident light interacts with the mixed phase medium at multi points at an interface of a surface of the multi-facet reflector and the mixed phase medium, as referred at step 516. Details of the propagation of light in the multi-facet reflector have been described earlier. The return journey of the incident light includes reflections, and attenuations received from the mixed phase medium at the multiple interaction points, which are collectively referred herein as collected light, from the multi-facet reflector. Step 518 refers to this collected light reaching and being received in an optical fiber.

At step 520, the method proceeds to detect electrical signals representative of collected light; and as shown at step 522, processing of these electrical signals is done to determine a characteristic output representative of composition of the mixed phase medium. Due to the use of more than one selectable wavelengths of light as incident light, the processing step is greatly simplified and does not require heavy empirical calculations used in the prior art methods. The detector output is directly matched in an exemplary processing step, with the incident light, and if attenuations of the concerned selected wavelength (from the selectable wavelength of incident light) are present, determination of corresponding phase is done, and characteristic output shown an indicator of presence of the concerned phase. Similarly, indicators are provided for presence or absence of each phase. When a phase is present, the corresponding detector output is further analyzed to know the concentration of the phase based on the intensity of the attenuated wavelength in the detector output. Finally, at step 524 the characteristic output is used for controlling the production of oil and gas by opening or closing the production valves and/or gas injection valves, to optimize the oil and gas output from the production well. In some implementations, the overall characteristic output obtained at different test sites in the same production well, may be used to determine the capacity of the production well, and accordingly proceed for operation or closure of the production.

As mentioned herein above the more than one selectable wavelengths include at least a first selectable wavelength substantially absorbed by oil, and substantially reflected by gas and water, and a second selectable wavelength substantially absorbed by water and substantially reflected by gas and oil.

The apparatus and method described herein offer the advantage of simultaneous incidence of more than one selectable wavelengths of light on the multi-facet reflector, and therefore in the mixed phase medium, and which ultimately leads to reducing complex post processing of the detector output required in the prior art embodiments. This feature enhances the sensitivity of collect light and therefore of the subsequent detector output. Therefore characteristic outputs provide clear discrimination of the three phases, gas, water, and oil present in the mixed phase medium.

The embodiments and aspects of invention referred herein above may analyze *in-situ*, the multiphase fluid in different types of flows, including flows in directional wells and horizontal wells. Further, the present apparatus and method described herein enable analysis of the multiphase fluid in a conduit or well in real-time, for example in milliseconds. Furthermore, the present apparatus and method analyze the multiphase fluid in a conduit or well without collecting a sample of the multiphase fluid outside or inside the conduit or well.

It would be understood by those skilled in the art that the different components of the apparatus 12 are implemented using processor, microprocessor, controller, general purpose processor of digital signal processor, based on the functional requirement of the components. The processing of data or signals is done in one example according to computer programs encoded with instructions on non-transitory computer readable medium There can also be memory coupled to the processors to store the computer programs, test results, analysis, characteristic outputs, as well as historical data. Further the processing can be done by the optical sensor probes alone, or in conjunction with the surface processing and display unit. Further, an integrated or a separate communication circuitry may be provided that is configured to transmit the signals and data between different components in apparatus 12.

The foregoing description is directed to particular embodiments of the present invention for the purpose of illustration and explanation. It will be apparent, however, to one skilled in the art that many modifications and changes to the embodiment set forth above are possible without departing from the scope of the invention, which is defined in claims 1 and 10. It is intended that the following claims be interpreted to embrace all such modifications and changes.

While only certain features of the invention have been illustrated and described herein, many modifications and changes will occur to those skilled in the art. It is, therefore, to be understood that the appended claims are intended to cover all such modifications and changes as fall within the scope of the invention, which is defined in claims 1 and 10.


## Claims

1. An optical sensor probe (26) for analyzing a composition of a mixed phase medium (14) in-situ at a test site (18) of a reservoir (16) for an oil and gas production well (20), the optical sensor probe comprising:

   an optical circuitry (42) for generating guided incident light (48) and for receiving collected light (54) from the mixed phase medium (14);
   a multi-facet reflector (56) in contact with the mixed phase medium (14) at the test site (18), for receiving the guided incident light (48) that, in operation, travels on a zig-zag path (50) in the multi-facet reflector (56) and impinges the mixed phase medium (14) at a plurality of interaction points (66), and for transmitting the collected light (54) received from the mixed phase medium (14) at the plurality of interaction points (66); and
   an optical fiber (52) coupled to the optical circuitry (42) and the multi-facet reflector (56), wherein the optical fiber (52), in operation, receives the guided incident light (48) from the optical circuitry (42), transmits the guided incident light (48) to the multi-facet reflector (56), receives the collected light (54) from the multi-facet reflector (56) and transmits the collected light (54) to the optical circuitry (42), wherein a principal axis (60) of the optical fiber (52) aligns substantially with a central axis (58) of the multi-facet reflector (56),
   wherein the optical circuitry (42) is configured to convert the collected light (54) into respective electrical signals and to process the respective electrical signals to generate a characteristic output (86) representing the composition of the mixed phase medium (14) at the test site (18),
   wherein the optical circuitry (42) is arranged to generate guided incident light comprising more than one selectable wavelengths (46) of light that, in operation, impinge the mixed phase medium (14) simultaneously, the more than one selectable wavelengths (46) comprising at least a first selectable wavelength of 350 nm to 450 nm substantially absorbed by oil, and substantially reflected by gas and water, and a second selectable wavelength of 1.2 $\mu$m to 3.3 $\mu$m substantially absorbed by water and substantially reflected by gas and oil, and
   wherein the multi-facet reflector (56) comprises:

      a cylinder (90) with a top end (92), a bottom end (94); and
      a cap (96) projecting from at least at the top end (92) of the cylinder (90), wherein the cap (96) has three or more non-similar faces (98, 100, 102) meeting at a common point (104), and at a predefined distance (106) from the top end (92) of the cylinder (90);
      wherein the cap (96) comprises a tiered structure with at least a first tier and a second tier, wherein a plurality of first faces (98, 100) are arranged as the first tier, and at least one second face (102), is arranged as a second tier, with the first tier being positioned below the second tier, and wherein at least one of the first faces (98,100) in the first tier is angularly positioned with respect to the second face (102) in the second tier.

2. The optical sensor probe (26) of claim 1, wherein the optical circuitry (42) comprises a light source (72) for generating more than one selectable wavelengths of light as incident light (48).

3. The apparatus (12) of claim 2, wherein the light source (72) is at least one of a coherent source, or an incoherent source.

4. The optical sensor probe (26) of claim 2 or claim 3, wherein the optical circuitry (42) comprises a modulator (76) for modulating the more than one selectable wavelengths of light at the light source (72).

5. The optical sensor probe (26) of any preceding claim, wherein the optical circuitry (42) comprises an optical coupler (70) to compensate for a difference in a numerical aperture of the optical fiber (52) and a numerical aperture of the multi-facet reflector (56).

6. The optical sensor probe (26) of any preceding claim, wherein the optical circuitry (42) comprises:

   a detector (78) for receiving the collected light (54) from the optical fiber (52), and for generating a detector output (82) representative of wavelengths present in the collected light (54); and
   a processor (84) for processing the detector output (82) and for generating a characteristic output (86) indicative of presence or absence or percent presence of each of gas, water and oil in the mixed phase medium (14).

7. The optical sensor probe (26) of any preceding claim, wherein the common point (104) lies on a central axis (58) of the cylinder (90).

8. The optical sensor probe (26) of any of claims 1 to 6, wherein the common point (104) is off-centered from the central axis (58) of the cylinder (90).

9. An apparatus (12) for analyzing a composition of a mixed phase medium (14) present in a reservoir (16) at a test site (18) of an oil and gas production well (20), wherein the mixed phase medium (14) comprises gas, water and oil, and wherein a wireline (24) is suspended to access the test site (18), the apparatus comprising:

   a plurality of optical sensor probes (26) of any one of the preceding claims suspended at one end of the wireline (24), wherein each optical sensor probe (26) is arranged to generate a characteristic output (86) representing the composition of the mixed phase medium (14) tested by the respective optical sensor probe (26); and
   a surface processing and display unit (28) connected at an other end of the wireline (24) for receiving the characteristic output (86) from each of the plurality of optical sensor probes (26), wherein the characteristic output (86) is used for controlling a production of the oil and gas production well.

10. A method of analyzing *in-situ* a composition of a mixed phase medium (14)
   present in a reservoir (16) at a test site (18) of an oil and gas production well (20), wherein the mixed phase medium comprises gas, water and oil, the method comprising:

   generating more than one selectable wavelengths of light;
   impinging the more than one selectable wavelengths of light simultaneously as guided incident light on a multi-facet reflector placed in the mixed phase medium, wherein the guided incident light is capable of travelling in a zig-zag path in the multi-facet reflector;
   transmitting the guided incident light through the multi-facet reflector, such that the guided incident light impinges the mixed phase medium at a plurality of interaction points travelling on the zig-zag path in a forward direction and a return direction;
   receiving collected light from the plurality of interaction points located on the multi-facet reflector;
   detecting electrical signals representative of the collected light; and
   processing the electrical signals representative of the collected light to determine a characteristic output representative of the composition of the mixed phase medium,
   wherein the more than one selectable wavelengths (46) comprise at least a first selectable wavelength of 350 nm to 450 nm substantially absorbed by oil, and substantially reflected by gas and water, and a second selectable wavelength of 1.2 $\mu$m to 3.3 $\mu$m substantially absorbed by water and substantially reflected by gas and oil; and
   wherein the multi-facet reflector (56) comprises:

a cylinder (90) with a top end (92), a bottom end (94); and
a cap (96) projecting from at least at the top end (92) of the cylinder (90), wherein the cap (96) has three or more non-similar faces (98, 100, 102) meeting at a common point (104), and at a predefined distance (106) from the top end (92) of the cylinder (90);
wherein the cap (96) comprises a tiered structure with at least a first tier and a second tier, wherein a plurality of first faces (98, 100) are arranged as the first tier, and at least one second face (102), is arranged as a second tier, with the first tier being positioned below the second tier, and wherein at least one of the first faces (98,100) in the first tier is angularly positioned with respect to the second face (102) in the second tier.

**Patentansprüche**

1. Optische Sensorsonde (26) zum Analysieren einer Zusammensetzung eines Mischphasenmediums (14) in-situ an einer Teststelle (18) eines Reservoirs (16) für eine Öl- und Gasförderbohrung (20), wobei die optische Sensorsonde umfasst:

   eine optische Schaltung (42) zum Erzeugen von geführtem einfallendem Licht (48) und zum Empfangen von gesammeltem Licht (54) aus dem Mischphasenmedium (14);
   einen Multi-Facettenreflektor (56) in Kontakt mit dem Mischphasenmedium (14) an der Teststelle (18) zum Empfangen des geführten einfallenden Lichts (48), das sich im Betrieb auf einem Zick-Zack-Pfad (50) in dem Multi-Facettenreflektor (56) ausbreitet und auf das Mischphasenmedium (14) an einer Vielzahl von Interaktionspunkten (66) auftrifft, und zum Übertragen des gesammelten Lichts (54), das von dem Mischphasenmedium (14) an der Vielzahl von Interaktionspunkten (66) empfangen wird; und
   eine optische Faser (52), die mit der optischen Schaltung (42) und dem Multi-Facettenreflektor (56) gekoppelt ist, wobei die optische Faser (52) im Betrieb das geführte einfallende Licht (48) von der optischen Schaltung (42) empfängt, das geführte einfallende Licht (48) zu dem Multi-Facettenreflektor (56) überträgt, das gesammelte Licht (54) von dem Multi-Facettenreflektor (56) empfängt und das gesammelte Licht (54) zu der optischen Schaltung (42) überträgt, wobei eine Hauptachse (60) der optischen Faser (52) im Wesentlichen auf eine Mittelachse (58) des Multi-Facettenreflektors (56) ausgerichtet ist,
   wobei die optische Schaltung (42) konfiguriert ist, um das gesammelte Licht (54) in jeweilige elektrische Signale umzuwandeln und die jeweiligen elektrischen Signale zu verarbeiten, um eine charakteristische Ausgabe (86) zu erzeugen, die die Zusammensetzung des Mischphasenmediums (14) an der Teststelle (18) darstellt,
   wobei die optische Schaltung (42) angeordnet ist, um geführtes einfallendes Licht zu erzeugen, das mehr als eine auswählbare Lichtwellenlänge (46) umfasst, die im Betrieb gleichzeitig auf das Mischphasenmedium (14) auftreffen, wobei die mehr als eine auswählbare Wellenlänge (46) mindestens eine erste auswählbare Wellenlänge von 350 nm bis 450 nm, die im Wesentlichen von Öl absorbiert und im Wesentlichen von Gas und Wasser reflektiert wird, und eine zweite auswählbare Wellenlänge von 1,2 bis 3,3 $\mu$m umfasst, die im Wesentlichen von Wasser absorbiert und im Wesentlichen von Gas und Öl reflektiert wird, und
   wobei der Multi-Facettenreflektor (56) umfasst:

      einem Zylinder (90) mit einem oberen Ende (92) und einem unteren Ende (94); und
      eine Kappe (96), die mindestens an dem oberen Ende (92) des Zylinders (90) vorsteht, wobei die Kappe (96) drei oder mehr nicht ähnliche Flächen (98, 100, 102) aufweist, die an einem gemeinsamen Punkt (104) und in einem vordefinierten Abstand (106) von dem oberen Ende (92) des Zylinders (90) aufeinandertreffen; wobei die Kappe (96) eine abgestufte Struktur mit mindestens einer ersten Ebene und einer zweiten Ebene umfasst, wobei eine Vielzahl von ersten Flächen (98, 100) als erste Ebene und mindestens eine zweite Fläche (102) als zweite Ebene angeordnet ist, wobei die erste Ebene unterhalb der zweiten Ebene positioniert ist und wobei mindestens eine der ersten Flächen (98, 100) in der ersten Ebene winklig in Bezug auf die zweite Fläche (102) in der zweiten Ebene positioniert ist.

2. Optische Sensorsonde (26) nach Anspruch 1, wobei die optische Schaltung (42) eine Lichtquelle (72) zum Erzeugen von mehr als einer auswählbaren Wellenlänge des Lichts als einfallendes Licht (48) umfasst.

3. Vorrichtung (12) nach Anspruch 2, wobei die Lichtquelle (72) mindestens eine kohärente Quelle oder eine inkohärente Quelle ist.

4. Optische Sensorsonde (26) nach Anspruch 2 oder Anspruch 3, wobei die optische Schaltung (42) einen Modulator (76) zum Modulieren der mehr als einen auswählbaren Wellenlänge des Lichts an der Lichtquelle (72) umfasst.

5. Optische Sensorsonde (26) nach einem der vorstehenden Ansprüche, wobei die optische Schaltung (42) einen optischen Koppler (70) umfasst, um eine Differenz zwischen einer numerischen Apertur der optischen Faser (52) und einer numerischen Apertur des Multi-Facettenreflektors (56) zu kompensieren.

6. Optische Sensorsonde (26) nach einem der vorstehenden Ansprüche, wobei die optische Schaltung (42) umfasst:

einen Detektor (78) zum Empfangen des gesammelten Lichts (54) von der optischen Faser (52) und zum Erzeugen einer Detektorausgabe (82), die für Wellenlängen repräsentativ ist, die in dem gesammelten Licht (54) vorhanden sind; und
einen Prozessor (84) zum Verarbeiten der Detektorausgabe (82) und zum Erzeugen einer charakteristischen Ausgabe (86), die das Vorhandensein oder Nichtvorhandensein oder das prozentuale Vorhandensein von jeweils Gas, Wasser und Öl in dem Mischphasenmedium (14) angibt.

7. Optische Sensorsonde (26) nach einem der vorstehenden Ansprüche, wobei der gemeinsame Punkt (104) auf einer Mittelachse (58) des Zylinders (90) liegt.

8. Optische Sensorsonde (26) nach einem der Ansprüche 1 bis 6, wobei der gemeinsame Punkt (104) exzentrisch von der Mittelachse (58) des Zylinders (90) angeordnet ist.

9. Vorrichtung (12) zum Analysieren einer Zusammensetzung eines Mischphasenmediums (14), das in einem Reservoir (16) an einer Teststelle (18) einer Öl- und Gasförderbohrung (20) vorhanden ist, wobei das Mischphasenmedium (14) Gas, Wasser und Öl umfasst und wobei eine Drahtleitung (24) aufgehängt ist, um auf die Teststelle (18) zuzugreifen, wobei die Vorrichtung umfasst:

eine Vielzahl von optischen Sensorsonden (26) nach einem der vorstehenden Ansprüche, die an einem Ende der Drahtleitung (24) aufgehängt sind, wobei jede optische Sensorsonde (26) so angeordnet ist, dass sie eine charakteristische Ausgabe (86) erzeugt, die die Zusammensetzung des von der jeweiligen optischen Sensorsonde (26) geprüften Mischphasenmediums (14) darstellt; und
eine Oberflächenverarbeitungs- und Anzeigeeinheit (28), die mit einem anderen Ende der Drahtleitung (24) verbunden ist, um die charakteristische Ausgabe (86) von jeder der Vielzahl von optischen Sensorsonden (26) zu empfangen, wobei die charakteristische Ausgabe (86) zum Steuern einer Produktion der Öl- und Gasförderbohrung verwendet wird.

10. Verfahren zum Analysieren *in-situ* einer Zusammensetzung eines Mischphasenmediums (14), das in einem Reservoir (16) an einer Teststelle (18) einer Öl- und Gasförderbohrung (20) vorhanden ist, wobei das Mischphasenmedium Gas, Wasser und Öl umfasst, wobei das Verfahren umfasst:

Erzeugen von mehr als einer auswählbaren Wellenlänge des Lichts;
gleichzeitiges Auftreffen des Lichts mit mehr als einer auswählbaren Wellenlänge als geführtes einfallendes Licht auf einen Multi-Facettenreflektor, der in dem Mischphasenmedium platziert ist, wobei das geführte einfallende Licht in der Lage ist, sich in einem Zick-Zack-Pfad in dem Multi-Facettenreflektor zu bewegen;
Übertragen des geführten einfallenden Lichts durch den Multi-Facettenreflektor, sodass das geführte einfallende Licht auf das Mischphasenmedium an einer Vielzahl von Interaktionspunkten auftrifft, die sich auf dem Zick-Zack-Pfad in einer Vorwärtsrichtung und einer Rückwärtsrichtung bewegen;
Empfangen von gesammeltem Licht von der Vielzahl von Interaktionspunkten, die sich auf dem Multi-Facettenreflektor befinden;
Erfassen von elektrischen Signalen, die für das gesammelte Licht repräsentativ sind; und
Verarbeiten der elektrischen Signale, die für das gesammelte Licht repräsentativ sind, um eine charakteristische Ausgabe zu bestimmen, die für die Zusammensetzung des Mischphasenmediums repräsentativ ist.
wobei die mehr als eine auswählbare Wellenlänge (46) mindestens eine erste auswählbare Wellenlänge von 350 nm bis 450 nm, die im Wesentlichen von Öl absorbiert und im Wesentlichen von Gas und Wasser reflektiert wird, und eine zweite auswählbare Wellenlänge von 1,2 $\mu$m bis 3,3 $\mu$m umfasst, die im Wesentlichen von Wasser absorbiert und im Wesentlichen von Gas und Öl reflektiert wird; und
wobei der Multi-Facettenreflektor (56) umfasst:

einem Zylinder (90) mit einem oberen Ende (92) und einem unteren Ende (94); und

eine Kappe (96), die mindestens an dem oberen Ende (92) des Zylinders (90) vorsteht, wobei die Kappe (96) drei oder mehr nicht ähnliche Flächen (98, 100, 102) aufweist, die an einem gemeinsamen Punkt (104) und in einem vordefinierten Abstand (106) von dem oberen Ende (92) des Zylinders (90) aufeinandertreffen; wobei die Kappe (96) eine abgestufte Struktur mit mindestens einer ersten Ebene und einer zweiten Ebene umfasst, wobei eine Vielzahl von ersten Flächen (98, 100) als erste Ebene und mindestens eine zweite Fläche (102) als zweite Ebene angeordnet ist, wobei die erste Ebene unterhalb der zweiten Ebene positioniert ist und wobei mindestens eine der ersten Flächen (98, 100) in der ersten Ebene winklig in Bezug auf die zweite Fläche (102) in der zweiten Ebene positioniert ist.

**Revendications**

1. Sonde de capteur optique (26) destinée à analyser une composition d'un milieu à phase mixte (14) in situ au niveau d'un site de test (18) d'un réservoir (16) pour un puits de production d'huile et de gaz (20), la sonde de capteur optique comprenant :

   un circuit optique (42) destiné à générer une lumière incidente guidée (48) et destiné à recevoir la lumière collectée (54) du milieu à phase mixte (14) ;
   un réflecteur multifacette (56) en contact avec le milieu à phase mixte (14) au niveau du site de test (18), destiné à recevoir la lumière incidente guidée (48) qui, lors du fonctionnement, se déplace sur un chemin en zigzag (50) dans le réflecteur multifacette (56) et heurte le milieu à phase mixte (14) au niveau d'une pluralité de points d'interaction (66), et destiné à transmettre la lumière collectée (54) reçue du milieu à phase mixte (14) au niveau de la pluralité de points d'interaction (66) ; et
   une fibre optique (52) couplée à la circuiterie optique (42) et au réflecteur multifacette (56), dans laquelle la fibre optique (52), lors du fonctionnement, reçoit la lumière incidente guidée (48) depuis la circuiterie optique (42), transmet la lumière incidente guidée (48) au réflecteur multifacette (56), reçoit la lumière collectée (54) du réflecteur multifacette (56) et transmet la lumière collectée (54) à la circuiterie optique (42), dans laquelle un axe principal (60) de la fibre optique (52) s'aligne sensiblement avec un axe central (58) du réflecteur multifacette (56),
   dans laquelle la circuiterie optique (42) est configurée pour convertir la lumière collectée (54) en signaux électriques respectifs et pour traiter les signaux électriques respectifs afin de générer une sortie caractéristique (86) représentant la composition du milieu à phase mixte (14) au niveau du site de test (18),
   dans laquelle la circuiterie optique (42) est agencée pour générer une lumière incidente guidée comprenant plus d'une longueur d'onde sélectionnable (46) de lumière qui, lors du fonctionnement, heurte simultanément le milieu à phase mixte (14), plus d'une longueur d'onde sélectionnable (46) comprenant au moins une première longueur d'onde sélectionnable de 350 à 450 nm sensiblement absorbée par l'huile, et sensiblement réfléchie par du gaz et de l'eau, et une seconde longueur d'onde sélectionnable de 1,2 à 3,3 $\mu$m sensiblement absorbée par l'eau et sensiblement réfléchie par du gaz et de l'huile, et
   dans laquelle le réflecteur multifacette (56) comprend :

      un cylindre (90) ayant une extrémité supérieure (92), une extrémité inférieure (94) ; et
      un capuchon (96) faisant saillie à partir d'au moins l'extrémité supérieure (92) du cylindre (90), dans laquelle le capuchon (96) a au moins trois faces non similaires (98, 100, 102) se rencontrant au niveau d'un point commun (104), et à une distance prédéfinie (106) de l'extrémité supérieure (92) du cylindre (90) ;
      dans laquelle le capuchon (96) comprend une structure étagée comportant au moins un premier étage et un second étage, dans laquelle une pluralité de premières faces (98, 100) sont agencées en tant que premier étage, et au moins une seconde face (102) est agencée en tant que second étage, le premier étage étant positionné en dessous du second étage, et dans laquelle au moins l'une des premières faces (98,100) dans le premier étage est positionnée angulairement par rapport à la seconde face (102) dans le second étage.

2. Sonde de capteur optique (26) selon la revendication 1, dans laquelle le circuit optique (42) comprend une source de lumière (72) destinée à générer plus d'une longueur d'onde sélectionnable de lumière en guise de lumière incidente (48).

3. Appareil (12) selon la revendication 2, dans lequel la source de lumière (72) est au moins l'une parmi une source cohérente, ou une source incohérente.

4. Sonde de capteur optique (26) selon la revendication 2 ou 3, dans laquelle la circuiterie optique (42) comprend un modulateur (76) destiné à moduler plus d'une longueur d'onde sélectionnable de lumière au niveau de la source de lumière (72).

5. Sonde de capteur optique (26) selon l'une quelconque des revendications précédentes, dans laquelle la circuiterie optique (42) comprend un coupleur optique (70) destiné à compenser une différence dans une ouverture numérique de la fibre optique (52) et une ouverture numérique du réflecteur multifacette (56).

6. Sonde de capteur optique (26) selon l'une quelconque des revendications précédentes, dans laquelle la circuiterie optique (42) comprend :

   un détecteur (78) destiné à recevoir la lumière collectée (54) de la fibre optique (52), et destiné à générer une sortie de détecteur (82) représentant des longueurs d'onde présentes dans la lumière collectée (54) ; et
   un processeur (84) destiné à traiter la sortie de détecteur (82) et destiné à générer une sortie caractéristique (86) indiquant la présence ou l'absence ou le pourcentage de présence de chacun parmi un gaz, de l'eau et de l'huile dans le milieu à phase mixte (14).

7. Sonde de capteur optique (26) selon l'une quelconque des revendications précédentes, dans laquelle le point commun (104) se situe sur un axe central (58) du cylindre (90).

8. Sonde de capteur optique (26) selon l'une quelconque des revendications 1 à 6, dans laquelle le point commun (104) est excentré de l'axe central (58) du cylindre (90).

9. Appareil (12) d'analyse d'une composition d'un milieu à phase mixte (14) présent dans un réservoir (16) au niveau d'un site de test (18) d'un puits de production d'huile et de gaz (20), dans lequel le milieu à phase mixte (14) comprend du gaz, de l'eau et de l'huile, et dans lequel un câble métallique (24) est suspendu pour accéder au site de test (18), l'appareil comprenant :

   une pluralité de sondes de capteur optique (26) selon l'une quelconque des revendications précédentes suspendues à une extrémité du câble (24), dans lequel chaque sonde de capteur optique (26) est agencée pour générer une sortie caractéristique (86) représentant la composition du milieu à phase mixte (14) testé par la sonde de capteur optique (26) respective ; et
   une unité de traitement de surface et d'affichage (28) connectée au niveau d'une autre extrémité du câble métallique (24) pour recevoir la sortie de caractéristique (86) de chacun parmi la pluralité de sondes de capteur optique (26), dans lequel la sortie de caractéristique (86) est utilisée pour commander une production du puits de production d'huile et de gaz.

10. Procédé d'analyse *in situ* d'une composition d'un milieu à phase mixte (14) présent dans un réservoir (16) au niveau d'un site de test (18) d'un puits de production d'huile et de gaz (20), dans lequel le milieu à phase mixte comprend du gaz, de l'eau et de l'huile, le procédé comprenant :

   la production de plus d'une longueur d'onde de lumière sélectionnable ;
   la production d'un impact sur plus d'une longueur d'onde sélectionnable de lumière simultanément en tant que lumière incidente guidée sur un réflecteur multifacette placé dans le milieu à phase mixte, dans lequel la lumière incidente guidée est capable de se déplacer sur un chemin en zigzag dans le réflecteur multifacette ;
   la transmission de la lumière incidente guidée à travers le réflecteur multifacette, de telle sorte que la lumière incidente guidée heurte le milieu à phase mixte au niveau d'une pluralité de points d'interaction se déplaçant sur le chemin en zigzag dans une direction vers l'avant et dans une direction de retour ;
   la réception de la lumière collectée provenant de la pluralité de points d'interaction situés sur le réflecteur multifacette ;
   la détection de signaux électriques représentant la lumière collectée ; et
   le traitement des signaux électriques représentant la lumière collectée pour déterminer une sortie caractéristique représentant la composition du milieu à phase mixte.
   dans lequel plus d'une longueur d'onde sélectionnable (46) comprend au moins une première longueur d'onde sélectionnable de 350 à 450 nm sensiblement absorbée par l'huile, et sensiblement réfléchie par du gaz et de l'eau, et une seconde longueur d'onde sélectionnable de 1,2 à 3,3 $\mu$m sensiblement absorbée par l'eau et sensiblement réfléchie par du gaz et de l'huile ; et
   dans lequel le réflecteur multifacette (56) comprend :

un cylindre (90) ayant une extrémité supérieure (92), une extrémité inférieure (94) ; et

un capuchon (96) faisant saillie à partir d'au moins l'extrémité supérieure (92) du cylindre (90), dans lequel le capuchon (96) a au moins trois faces non similaires (98, 100, 102) se rencontrant au niveau d'un point commun (104), et à une distance prédéfinie (106) de l'extrémité supérieure (92) du cylindre (90) ;

dans lequel le capuchon (96) comprend une structure étagée comportant au moins un premier étage et un second étage, dans lequel une pluralité de premières faces (98, 100) sont agencées en tant que premier étage, et au moins une seconde face (102) est agencée en tant que second étage, le premier étage étant positionné en dessous du second étage, et dans lequel au moins l'une des premières faces (98,100) dans le premier étage est positionnée angulairement par rapport à la seconde face (102) dans le second étage.

FIG. 1

FIG. 2

FIG. 3

FIG. 4

FIG. 5

FIG. 6

FIG. 7

500

| Generating more than one selectable wavelengths of light with different modulation frequency | 512 |

| Impinging the more than one selectable wavelengths of light simultaneously as guided incident light on a multi-facet reflector placed in a mixed phase medium, where the guided incident light is capable of travelling in a triangular path in a multi-facet reflector | 514 |

| Transmitting the guided incident light through the multi-facet reflector, such that the guided incident light impinges the mixed phase medium at a plurality of interaction points travelling on the triangular path in a forward direction and a return direction | 516 |

| Receiving collected light from the multi-facet reflector from the plurality of interaction points | 518 |

| Detecting electrical signals representative of collected light | 520 |

| Processing electrical signals with frequency multiplexed lock in heterodyne demodulation technique representative of the collected light to determine a characteristic output representative of composition of the mixed phase medium | 522 |

| Using the characteristic output for controlling production from oil and gas production well | 524 |

FIG. 8

## REFERENCES CITED IN THE DESCRIPTION

**Patent documents cited in the description**

- US 2015027243 A1
- US 2016164125 A1
- US 2007120051 A1
- US 2002176646 A1
- US 2012170023 A1
- WO 2016044005 A1